# EUROPEAN PATENT APPLICATION

(11) **EP 1 190 707 A1**
(43) Date of publication of application: **27.03.2002**
(21) Application number: 01830098.8
(22) Date of filing: 14.02.2001
(51) Int. Cl.: A61K 9/70, A61K 47/14, A61K 47/08

(54) **High penetration transdermal medicament**

(30) Priority: 22.09.2000 IT MI002075
(71) Applicant: Ciocca, Gianluigi, 20137 Milano (MI) (IT)
(72) Inventor: Ciocca, Gianluigi, 20137 Milano (MI) (IT)
(74) Representative: Pizzoli, Antonio

(57) **Abstract**

The present invention relates to a transdermal medicament containing a penetration accelerator selected from the group consisting of:
a) one or more methyl esters of a saturated or unsaturated monocarboxylic acid having 8 to 12 carbon atoms; and/or
b) one or more ethers selected from the group comprising methyl-, ethyl- and β-hydroxyethylethers of saturated aliphatic alcohols having 8 to 12 carbon atoms,
   as well as:
   a medicated plaster or a therapeutical plaster containing said transdermal medicament.

## Description

The present invention relates to a transdermal medicament releasing medicinal substances throughout the skin.

As an example of such a kind of medicament, we may mention, by way of illustration:
- transdermal plasters containing nicotine for fighting smoke pharmacodependency (see e.g. EP0289342);
- transdermal plasters for hormonal therapies, containing for instance estradiol (see e.g. EP0275716, EP0285563, EP0689458 and EP0802789);
- the so called medicated plasters, obtainable for instance from The Boots Company, containing the flurbiprofen anti-inflammatory.

In order to intensify the penetration of a drug through the skin, Elan Transdermal (EP0289342) recommended the use of a few aprotic solvents, such as dimethylsulfoxide, N,N-dimethylformamide, N,N-dimethylacetamide and N-methylpyrrolidone.

In the last years, LTS Lohman Therapie-Systeme (EP0689458) proposed using a penetration accelerator selected from mono- and di-isopropylidenglycerol and possibly adding a penetration adjuvant, having a glycol or pyrrolidone nature.

The same LTS, in more recent times (EP0802789) suggested to enhance the penetration rate of estradiol through the skin by adding lauric acid, in the presence of an adhesive substance based on cellulose derivatives.

It is an object of the present invention to improve a transdermal medicament from both the therapeutical and economical point of view, by incorporating therein a penetration accelerator being on the one hand effective and safe and on the other hand cheap and available without particular problems.

Such an object can be easily reached by incorporating in a transdermal medicament a penetration accelerator selected from the group consisting of:
a) one or more methyl esters of a saturated or unsaturated monocarboxylic aliphatic acid having 8 to 12 carbon atoms; and/or
b) one or more ethers selected from the group comprising methyl-, ethyl- and β-hydroxyethylethers of saturated aliphatic alcohols having 8 to 12 carbon atoms.

As a saturated or unsaturated monocarboxylic acid, mention may be made for instance of octanoic, decanoic, undecenoic and lauric acid.

As a saturated aliphatic alcohol, mention may be made for instance of n-octyl, 2-ethylhexyl, decyl and lauryl alcohol.

Particularly advantageous results can be reached by employing the methyl ester of undecenoic acid and/or the β-hydroxyethylethers of n-octyl and/or 2-ethylhexyl and/or decyl and/or lauryl alcohol and/or the methyl ether of lauryl alcohol.

The addition of said esterifying or etherifying end group can increase the penetration rate of a transdermal drug, without having recourse to the expensive levulinic acid, optionally in the form of a supercooled liquid, disclosed in EP0792145.

## Claims

1. Transdermal medicament containing a penetration accelerator selected from the group consisting of:
a) one or more methyl esters of a saturated or unsaturated monocarboxylic aliphatic acid having 8 to 12 carbon atoms; and/or
b) one or more ethers selected from the group comprising methyl-, ethyl- and β-hydroxyethylethers of saturated aliphatic alcohols having 8 to 12 carbon atoms.

2. Transdermal medicament according to claim 1, wherein said saturated or unsaturated monocarboxylic acid is selected from octanoic, decanoic, undecenoic and lauric acid.

3. Transdermal medicament according to claim 1, wherein said one or more ethers are selected from the β-hydroxyethylethers of n-octyl, 2-ethylhexyl, decyl and lauryl alcohol.

4. Transdermal medicament according to claim 1, wherein said ester and/or ether is selected from the group comprising the methyl ester of undecenoic acid, the β-hydroxyethylethers of claim 3 and the methyl ether of lauryl alcohol.

5. Medicated plaster comprising the transdermal medicament according to any of the claims 1 to 4.

6. Therapeutical plaster comprising the transdermal medicament according to any of the claims 1 to 4.

7. The therapeutical plaster of claim 6, further comprising nicotine.

8. The therapeutical plaster of claim 6, further comprising estradiol.
